## Europäisches Patentamt
## European Patent Office
## Office européen des brevets

(11) Veröffentlichungsnummer: **0 214 311**
**A1**

## (12) EUROPÄISCHE PATENTANMELDUNG

Veröffentlicht nach Art. 158 Abs. 3 EPÜ

(21) Anmeldenummer: **86902585.8**

(22) Anmeldetag: **06.03.86**

Daten der zugrundeliegenden internationalen Anmeldung:

(86) Internationale Anmeldenummer:
**PCT/SU86/00020**

(87) Internationale Veröffentlichungsnummer:
**WO86/05073 (12.09.86 86/20)**

(51) Int. Cl.⁴: **A 23 J 1/18**
**C 12 N 1/16**

(30) Priorität: **06.03.85 SU 3865414**
**20.06.85 SU 3938704**
**13.08.85 SU 3944226**

(43) Veröffentlichungstag der Anmeldung:
**18.03.87 Patentblatt 87/12**

(84) Benannte Vertragsstaaten:
**BE CH DE FR GB IT LI NL SE**

(71) Anmelder: **BELORUSSKY GOSUDARSTVENNY UNIVERSITET IMENI V.I.LENINA**
**Leninsky pr., 4**
**Minsk, 220080(SU)**

(72) Erfinder: **VARGANOV, Vladimir Alexandrovich**
**ul. Miroshnichenko, 53-59**
**Minsk, 220135(SU)**

(72) Erfinder: **PINCHUK, Grigory Petrovich ul. Zhukovskogo, 7-3**
**Minsky raion Minskaya obl.**
**derevnya Schomyslitsa, 223049(SU)**

(72) Erfinder: **POLUNCHENKO, Vitaly Vasilievich**
**ul. Kakhovskaya, 38-34**
**Minsk, 220068(SU)**

(72) Erfinder: **GOVORUN, Alexandr Konstantinovich**
**ul. Bobruiskaya, 21-9**
**Minsk, 220050(SU)**

(72) Erfinder: **GORBACH, Anton Efimovich ul.**
**Komsomolskaya, 6-34**
**Gomelskaya obl.**
**Kalinkovichi, 247710(SU)**

(72) Erfinder: **KRJUKOV, Anatoly Fedorovich**
**ul. Landera, 20-88**
**Minsk, 220046(SU)**

(74) Vertreter: **Lehn, Werner, Dipl.-Ing. et al,**
**Hoffmann, Eitle & Partner Patentanwälte Arabellastrasse 4 (Sternhaus)**
**D-8000 München 81(DE)**

(54) **HERSTELLUNGSVERFAHREN EINES PROTEINVITAMINPRÄPARATS.**

(57) A method of obtaining a protein-vitaminous concentrate consists in cultivating yeast in the aerobic conditions on a nutrient medium consisting of effluents from meat-packing plants containing, by mass, not less than 0.01 per cent of lipids, 0.01 per cent of protein components, 0.005 per cent of carbohydrates with an addition of nitrogen – and phosphorus – containing mineral salts at the amount of not less than 0.1 per cent by mass in relation to the volume of the effluents, after which the biomass is extracted and the desired product is obtained.

# VERFAHREN ZUR HERSTELLUNG EINES EIWEIß-VITAMIN-KONZENTRATES

## Gebiet der Technik

Die vorliegende Erfindung bezieht sich auf die mikrobiologische Industrie und betrifft insbesondere ein Verfahren zur Herstellung eines Eiweiß-Vitamin-Konzentrates, welches als Futterzusatz in der Viehzucht zur Verwendung kommt sowie als Quelle für Aminosäuren, verschiedene Lipide, Vitamine Verwendung finden kann, wobei die letztgenannten in der mikrobiologischen, pharmazeutischen, Parfümindustrie und in anderen Industriezweigen eingesetzt werden können. Das erfindungsgemäße Verfahren läßt sich außerdem zur Aufbereitung von in Fleischkombinaten anfallenden Abwässern verwenden.

## Vorheriger Stand der Technik

Bekannt sind verschiedene Verfahren zur Herstellung eines Eiweiß-Vitamin-Konzentrates durch Kultivierung von Hefen auf synthetischen mineralischen Nährböden, die Kohlenhydrate oder Kohlenwasserstoffe enthalten, unter Belüftungsbedingungen. (Biotechnologie der mikrobiellen Synthese, Riga, Verlag Sinatne, 1980, S. 12 bis 43; Biotekhnologia mikrobnogo sinteza, Riga, Izdatelstvo Zinatne, 1980, S. 12-43).

Die Herstellung von synthetischen Nährböden, die in den angegebenen Verfahren verwendet werden, ist mit der Durchführung mehrerer technologischer Arbeitsgänge und zwar Zubereitung der Konzentrate von Makro- und Mikronährstoffen, ihre Auflösung, vorherige Behandlung einer Kohlenstoffquelle und Eintragen derselben in den Nährboden verbunden. Kohlenstoffquellen wie n-Alkane sind praktisch in wäßriger Phase unlöslich, und es gibt deshalb eine Reihe von Schwierigkeiten, verbunden nicht nur mit der Herstellung eines Nährbodens für Mikroorganismen, sondern auch mit der Verwirklichung des Kultivierungsprozesses, insbesondere Probleme des Emulgierens von n-Alkanen, der getrennten Zufhr des Nährbodens selbst und einer Kohlenstoffquelle, der Verstärkung von Belüftungs-und Vermischungsverhältnissen. Da die Wachstumsgeschwindigkeit von Hefen auf n-Alkanen etwa 3-bis 3,5mal so klein wie auf Kohlenwasserstoffen ist, so sind dementsprechend die Stoffaustauschdaten der Fermentationsausrüstung ebensoviel zu erhöhen. Industriell kann man außerdem das Verfahren zur Herstellung einer hefigen Biomasse auf n-Alkanen (im Durchfluß und kontinuierlich) bei einer über 0,20 bis 0,25 St. liegenden Durchflußgeschwindigkeit praktisch nicht verwirklichen. Für die angegebenen Verfahren ist ein kleiner Eiweißgehalt in Eiweiß-Vitamin-Konzentraten, der 50 bis 60 % nicht übersteigt, charakteristisch.

Bekannt ist auch ein Verfahren zur Herstellung eines Eiweiß-Vitamin-Konzentrats durch Kultivierung von Hefen auf einem Nährboden, enthaltend Kohlenstoffquellen, Mineralstoffe, Wuchsstoffe, unter Belüftungsbedingungen. Als Nährboden dient eine Molke. (SU-Urheberschein Nr. 571339, IPK C12 N, 1976). Die Molke stellt ein flüssiges Abfallprodukt dar, welches bei der Herstellung von Sauermilcherzeugnissen anfällt, und enthält bedeutende Mengen von Laktose, die bei diesem Verfahren als Kohlenstoffquelle für Hefen dient.

Die Verwendung der Molke als Nährboden macht es erforderlich, in der Molke praktisch fehlende Mineralsalze zusätzlich einzuführen. Der Laktosegehalt der Molke ist außerdem gering, und zwecks Durchführung des Verfahrens ist die Molke vorher einzudicken, um die Laktosekonzentration zu steigern. Molkeneiweiße, die das intensive Wachstum von Hefen stören, werden im voraus ausgezogen. Alle genannten Arbeitsgänge machen die Technologie zur Herstellung des Eiweiß-Vitamin-Konzentrats kompliziert. Der Nachteil dieses Verfahrens besteht auch darin, daß der Nährboden auf der Molkenbasis selbst bei der schwach intensiven Belüftung zum Schäumen geneigt ist, was sich nicht nur auf der Aktivität des mikrobiologischen Prozesses, sondern auch auf der Produktionskultur negativ auswirkt.

- 3 -

Die ziemlich komplizierte und energieintensive vorherige Bereitung des Nährbodens gestattet die Kultivierung von Hefen praktisch nur diskontinuierlich durchzuführen, wodurch die Effektivität bei der Herstellung der hefigen Biomasse herabgesetzt wird. Das hergestellte Eiweiß-Vitamin-Konzentrat enthält höchstens 50 bis 55 % Eiweiß.

Darlegung der Erfindung

Der Erfindung liegt die Aufgabe zugrunde, durch Änderung des Nährbodens ein Verfahren zu entwickeln, welches es ermöglicht, die Technologie der Herstellung eines Eiweiß-Vitamin-Konzentrates zu vereinfachen und zu verbilligen, seine Eigenschaften als Futtermittel zu verbessern sowie die Abwasserverwertung und -reinigung gleichzeitig zu verwirklichen.

Die Aufgabe wird dadurch gelöst, daß man in einem Verfahren zur Herstellung eines Eiweiß-Vitamin-Konzentrates durch Kultivierung von Hefen auf einem Nährboden, welcher Kohlenstoffquellen, Mineralstoffe und Wuchsstoffe enthält, unter Belüftungsbedingungen, anschließende Trennung der Biomasse und darauffolgende Herstellung des Endprodukts erfindungsgemäß als Nährboden in Fleischkombinaten anfallende Abwässer benutzt, welche mindenstens 0,01 Masse% Lipide, 0,01 Masse% Eiweißstoffe, 0,005 Masse% Kohlenhydrate unter Zusatz stickstoff- und phosphorhaltiger Mineralsalze in einer Menge von mindenstens 0,1 Masse%, bezogen auf Abwasservolumen, enthalten.

Als stickstoff- und phosphorhaltige Mineralsalze verwendet man vorzugsweise Ammoniumphosphat.

Zur Steigerung der Ausbeute an Biomasse werden n-Alkane in einer Menge von 0,1 bis 4 Masse%, bezogen auf das Nährbodenvolumen, in den Nährboden eingetragen. Als Wuchsstoffe für Hefen ist es zweckmäßig, die Vollgalle in einer Menge von 0,1 bis 0,0005 Masse%, bezogen auf das Nährbodenvolumen, in den Nährboden zusätzlich einzutragen.

Das erfindungsgemäße Verfahren ermöglicht die Verwendung von Produktionsabfällen und zwar von in Fleischkombinaten anfallenden Abwässern als Nährboden, was ökonomisch vorteilhaft ist und die Verfahrenstechnologie vereinfacht.

Das erfindungsgemäße Verfahren bietet die Möglichkeit, ein Eiweiß-Vitamin-Konzentrat in einer hohen Ausbeute und mit hohem Gehalt an biologisch wertvollen Substanzen herzustellen. Das erfindungsgemäße Verfahren sichert die gleichzeitige Abwasserverwertung und -reinigung.

Die beste Ausführungsform des Verfahrens

Das erfindungsgemäße Verfahren zur Herstellung eines Eiweiß-Vitamin-Konzentrates wird wie folgt verwirklicht.

Zu im Fleischkombinat anfallenden Abwässern, welche mindenstens 0,01 Masse% Lipide, 0,01 Masse% Eiweißstoffe, 0,005 Masse% Kohlenhydrate enthalten, gibt man stickstoff- und phosphorhaltige Mineralsalze in einer Menge von mindenstens 0,1 Masse%, bezogen auf das Abwasservolumen, trägt ein Hefeinokulum ein und verwirklicht die Kultivierung von Hefen unter Belüftung bei Temperaturen und pH-Werten, welche für die gewählte Hefekultur günstig sind.

Als stickstoff- und phosphorhaltige Mineralsalze benutzt man vorteilhaft Ammoniumphosphat.

Als Inokulum können beliebige Hefen zum Beispiel der Gattungen Candida, Saccharomyces zur Verwendung kommen.

Ihre Kultivierung erfolgt periodisch oder kontinuierlich im Durchfluß unter Belüftungsbedingungen. Als zusätzliche Kohlenstoffquelle werden im Bedarfsfall und abhängig von der entsprechenden Wahl der Hefekultur n-Alkane in einer Konzentration von 0,1 bis 4 Masse%, bezogen auf das Nährbodenvolumen, und als Wuchsstoff die Vollgalle in einer Menge von 0,0005 bis 0,1 Masse%, bezogen auf das Nährbodenvolumen, eingetragen. Durch völlige Verwertung der Kohlenstoffquellen trennt man die hefige Biomasse ab und erhält ein Eiweiß-Vitamin-Konzentrat, welches in flüssigem, pastenartigem und getrocknetem Zustand den Tieren und dem Geflügel verfüttert wird oder nach der angemessenen Verarbeitung in den unterschiedlichen Industriezweigen eingesetzt wird. Der technologische Prozeß der Herstellung eines Eiweiß-Vitamin-Präparates nach dem erfindungsgemäßen Verfahren weist eine Reihe von Vorteilen gegenüber den bekannten Verfahren auf. Als Basis des Nährbodens dienende Abwässer, die in Fleischkombinaten anfallen, stellen einen Produktionsabfall dar und sind ökonomisch vorteilhaft. Dadurch, daß man dem Nährboden eine einzige Quelle für Stickstoff und Phosphor und eine zusätzliche Kohlenstoffquelle in Form von n-Alkanen oder die Vollgalle als Wuchsstoff zugibt, wird die Ausbeute an Biomasse erhöht. Im technologischen Prozeß kommt also der Nährboden zum Einsatz, welcher keine vorherige Behandlung erfordert, ökonomisch vorteilhaft ist, die hefige Biomasse in hoher Ausbeute herzustellen ermöglicht, welche große Mengen von Eiweiß und anderen biologisch wichtigen Bestandteilen enthält. Da die Hefen je nach ihrem Wachstum organische Komponenten von in Fleischkombinaten anfallenden Abwässern verwerten, kann man nach dieser Technologie auch die gleichzeitige Abwasserreinigung durchführen. Da die Kultivierungsbedingungen für Hefen erniedrigte pH-Werte vorsehen, die für die Entwicklung der Mikroflora ungünstig sind, so wird die Verwendung dieser Technologie dazu beitragen, daß der Bakterienbesatz in durch Fermentation anfallenden Abflüssen verringert wird, Da in in Fleischkombinaten anfallenden Abwassern natürliche hochwertige Kohlenstoffquellen enthalten sind, ist die biochemische Zusammensetzung der hefigen Biomasse von größerem Wert als die nach den bekannten Verfahren hergestellte Biomasse. Der Gesamtgehalt dieser Biomasse an Eiweiß und Lipiden beträgt zum Beispiel etwa 78 bis 80 %, bezogen auf die Trockenmasse, während dieser Kennwert im Falle der Kultivierung von Hefen auf Kohlenhydraten oder Kohlenwasserstoffen 70 bis 72% nicht übersteigt. Das erhältbare Eiweiß-Vitamin-Präparat wird außerdem keine Beschränkungen in der Verwendung hinsichtlich toxikologischer Anforderungen haben.

Zum besseren Verstehen der vorliegenden Erfindung werden folgende Durchführungsbeispiele des Verfahrens zur Herstellung eines Eiweiß-Vitamin-Konzentrates angeführt.

Beispiel 1

Zu 1 Liter in einem Fleischkombinat anfallendem Abwasser, enthaltend 0,1 Masse% Lipide, 0,05 Masse% Eiweißstoffe und 0,005 Masse% Kohlenhydrate, setzt man 0,2 Masse% Ammoniumphosphat und 0,01 Masse% (bezogen auf die Trockenmasse) Hefe Candida sp. zu. Die Kultivierung erfolgt unter Belüftung bei einer zwischen 28 und 30°C liegenden Temperatur und einem pH-Wert von 4,0 bis 4,2 in einem Fermenter diskontinuierlich unter keiner Sterilität innerhalb von 3 Stunden.

Nach der Beendigung der Kultivierung wird die Biomasse abgetrennt und getrocknet. Die Ausbeute an trockenem Eiweiß-Vitamin-Konzentrat beträgt 1,28 g/l, wobei es 69,3 Masse% Eiweiß und 130,8 mg/kg Riboflavin enthält.

Beispiel 2

Zu 1 Liter in einem Fleischkombinat anfallendem Abwasser, enthaltend 1,5 Masse% Lipide, 0,3 Masse% Eiweißstoffe und 0,007 Masse% Kohlenhydrate, setzt man 0,2 Masse% Ammoniumphosphat und 0,05 Masse% (Hefe Candida sp. (bezogen auf die Trockenmasse) zu. Die Kultivierung erfolgt unter Belüftung bei einer zwischen 30 und 32°C liegenden Temperatur und einem pH-Wert von 4,2 bis 4,5 in einem Fermenter diskontinuierlich unter keiner Sterilität innerhalb von 7 Stunden.

Nach der Beendigung der Kultivierung wird die Biomasse abgetrennt und getrocknet. Die Ausbeute an trockenem Eiweiß-Vitamin-Konzentrat beträgt 16,93 g/l, wobei es 75,21 Masse% Eiweiß und 340,7 mg/kg Riboflavin enthält.

Beispiel 3

Auf der Basis von in einem Fleischkombinat anfallendem Abwasser, enthaltend 1,0 Masse% Lipide, 0,2 Masse% Eiweißstoffe, 0,005 Masse% Kohlenhydrate, worin 0,2 Masse% Ammoniumphosphat eingetragen werden, wird die aerobe Kultivierung von Hefe Candida sp. im Durchfluß bei einer Durchflußgeschwindigkeit von 0,5 St. $^{-1}$ durchgeführt.

Die Kultivierung erfolgt bei einer zwischen 30 und 32°C liegenden Temperatur und einem pH-Wert von 4,0 bis 4,2. Die Prozeßproduktivität beträgt 5,83 g/l. St. Eiweiß-Vitamin-Konzentrat (Trockenmasse), wobei es 74,92 Masse% Eiweiß und 321,5 mg/kg Riboflavin enthält.

Beispiel 4

Auf der Basis von in einem Fleischkombinat anfallendem Abwasser, enthaltend 1,0 Masse% Lipide, 0,2 Masse% Eiweißstoffe, 0,012 Masse% Kohlenhydrate, worin 0,1 Masse% Ammoniumphosphat eingetragen werden, wird die aerobe Kultivierung von Hefe Saccharomyces sp. im Durchfluß durchgeführt. Die Kultivierung erfolgt bei einer zwischen 28 und 32°C. liegenden Temperatur und einem pH-Wert von 4,0 bis 4,2 bei einer Durchflußgeschwindigkeit von 0,45 St. $^{-1}$. Die Prozeßproduktivität beträgt 5,03 g/l.St. Eiweiß-Vitamin-Konzentrat (Trockenmasse), wobei es 73,3 Masse% Eiweiß und 312 mg/kg Riboflavin enthält.

Beispiel 5

Zu 1 Liter in einem Fleischkombinat anfallendem Abwasser, enthaltend 0,09 Masse% Lipide, 0,09 Masse% Eiweißstoffe, 0,61 Masse% Kohlenhydrate, setzt man 0,2 Masse% Ammoniumphosphat, 0,01 Masse% Vollgalle und 1 g Hefe Saccharomyces sp. (75%iger Feuchtigkeit) zu. Die Kultivierung erfolgt auf einem Schütteltisch unter Belüftungsbedingungen diskontinuierlich bei einer Temperatur von 30°C und einem pH-Wert von 4,2 innerhalb von 4 Stunden. Die Ausbeute an trockenem Eiweiß-Vitamin-Konzentrat beträgt 5,36 g/l, wobei es 71,8 Masse% Eiweiß und 235,6 mg/kg Riboflavin enthält.

Beispiel 6

Der Prozeß erfolgt analog zu dem im Beispiel 5

- 8 -

beschriebenen. In den Nährboden werden 0,0005 Masse% Vollgalle eingetragen. Die Ausbeute an trockenem Eiweiß-Vitamin-Konzentrat beträgt 4,2 g/l, wobei es 70,7 Masse% Eiweiß und 226,8 mg/kg Riboflavin enthält.

Beispiel 7

Man bereitet einen Nährboden zur Kultivierung von Hefen auf der Basis von in einem Fleischkombinat anfallendem Abwasser, enthaltend 1,0 Masse% Fett, 0,08 Masse% Eiweißstoffe, 0,005 Masse% Kohlenhydrate, zu. Dem Nährboden werden 3,0 Masse% n-Alkane (Mischung von Fraktionen mit 12 bis 18 C-Atomen) und 0,3 Masse% Ammoniumsulfat, 0,08 Masse% Kaliumphosphat (monosubstituiert), 0,03 Masse% Natriumphosphat (disubstituiert) zusätzlich zugegeben. Hefe Candida sp. wird in einer Menge von 3 g/l (bezogen auf die Trockenmasse) eingetragen und in einem Fermenter unter Belüftungsbedingungen innerhalb von 12 Stunden bei einer zwischen 32 und 34°C liegenden Temperatur und einem pH-Wert von 4,0 bis 4,2 kultiviert. Die Ausbeute an trockenem Eiweiß-Vitamin-Konzentrat beträgt 37,8 g/l, wobei es 75,3 Masse% Eiweiß und 396,3 mg/kg enthält.

Beispiel 8

Der Prozeß erfolgt analog zu dem im Beispiel 7 beschriebenen. In den Nährboden werden 0,02 Masse% Vollgalle eingetragen. Die Ausbeute an trockenem Eiweiß-Vitamin-Konzentrat beträgt 40,5 g/l, wobei es 74,8 Masse% Eiweiß und 350,0 mg/kg Riboflavin enthält.

PATENTANSPRÜCHE

1. Verfahren zur Herstellung eines Eiweiß-Vitamin-Konzentrates durch Kultivierung von Hefen auf einem Nährboden, enthaltend Kohlenstoffquellen, Mineralstoffe und Wuchsstoffe, unter Belüftungsbedingungen, anschließende Trennung der Biomasse und darauffolgende Herstellung des Endprodukts, d a d u r c h   g e k e n n z e i c h - n e t , daß man als Nährboden in Fleischkombinaten anfallende Abwässer verwendet, welche mindenstens 0,01 Masse% Lipide, 0,01 Masse% Eiweißstoffe, 0,005 Masse% Kohlenhydrate unter Zusatz stickstoff- und phosphorhaltiger Mineralsalze in einer Menge von mindestens 0,1 Masse%, bezogen auf das Abwasservolumen, enthalten.

2. Verfahren nach Anspruch 1, d a d u r c h   g e k e n n z e i c h n e t , daß als stickstoff- und phosphorhaltige Mineralsalze Ammoniumphosphat verwendet wird.

3 . Verfahren nach Ansprüchen 1 bis 2, d a d u r c h   g e k e n n z e i c h n e t , daß dem Nährboden n-Alkane in einer Menge von 0,1 bis 4 Masse%, bezogen auf das Nährbodenvolumen, zusätzlich eingetragen werden.

4. Verfahren nach Ansprüchen 1 bis 3, d a d u r c h   g e k e n n z e i c h n e t , daß als Wuchsstoff dem Nährboden die Vollgalle in einer Menge von 0,1 bis 0,0005 Masse%, bezogen auf das Nährbodenvolumen, zusätzlich eingetragen wird.

# INTERNATIONAL SEARCH REPORT

International Application No PCT/SU 86/00020

**I. CLASSIFICATION OF SUBJECT MATTER** (if several classification symbols apply, indicate all) *

According to International Patent Classification (IPC) or to both National Classification and IPC

IPC⁴ − A23J 1/18, C12N 1/16

**II. FIELDS SEARCHED**

Minimum Documentation Searched [7]

| Classification System | Classification Symbols |
|---|---|
| IPC⁴ | A23J 1/18, C12N 1/16, C12C 11/18 |

Documentation Searched other than Minimum Documentation
to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US . A. 3966554. (Novex RT). 29 June 1976 (29.06.76).see the claims, examples 1−5 | 1 |
| A | GB, A, 1407516 (Novex RT), 24 September 1975 (24.09.75), see the claims | 1 |
| A | GB, A, 1471921 ( AJINOMOTO CO., INC.), 27 April 1977 (27.04.77), see the claims | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 22 May 1986 (22.05.86) | 03 June 1986 (03.06.86) |
| International Searching Authority | Signature of Authorized Officer |
| ISA/SU | |

Form PCT/ISA/210 (second sheet) (January 1985)